Europäisches Patentamt

European Patent Office

Office européen des brevets

(11) Numéro de publication: **0 347 267 B1**

(12) **FASCICULE DE BREVET EUROPEEN**

(45) Date de publication de fascicule du brevet: **16.03.94**   (51) Int. Cl.⁵: **A61L 27/00**, C08L 33/20

(21) Numéro de dépôt: **89401218.6**

(22) Date de dépôt: **28.04.89**

(54) **Hydrogel non réticulé, son procédé de préparation, son application en médecine ou en chirurgie, notamment comme implant oculaire.**

(30) Priorité: **02.05.88 FR 8805856**

(43) Date de publication de la demande:
**20.12.89 Bulletin 89/51**

(45) Mention de la délivrance du brevet:
**16.03.94 Bulletin 94/11**

(84) Etats contractants désignés:
**BE DE ES FR GB GR IT LU NL**

(56) Documents cités:
**DE-A- 2 028 956**
**DE-A- 2 508 416**
**US-A- 4 731 079**

(73) Titulaire: **INSTITUT NATIONAL DE LA SANTE ET DE LA RECHERCHE MEDICALE (INSERM)**
**101, rue de Tolbiac**
**F-75654 Paris Cédex 13(FR)**

(72) Inventeur: **Honiger, Jiri**
**14 rue Louise Michel**
**F-94800 Villejuif(FR)**
Inventeur: **Laroche, Laurent**
**119 rue des Pyrénées**
**F-75020 Paris(FR)**

(74) Mandataire: **Kerneis, Danièle**
**Service Brevets**
**HOSPAL R & D Int.**
**BP 21**
**F-69881 Meyzieu Cédex (FR)**

EP 0 347 267 B1

## Description

La présente invention est relative à un hydrogel non réticulé, à son procédé de préparation ainsi qu'à ses applications comme objet à usage médical et/ou chirurgical tels que tubes, fils, films, joncs, implants et analogues, notamment en ophtalmologie.

On a déjà proposé, de mettre en oeuvre des polymères sous forme d'hydrogels présentant un contenu en eau relativement élevé tout en ayant des propriétés mécaniques et optiques améliorées (Brevet européen n° 188 110 et Brevets américains 4 379 864 et 4 543 371), pour des applications ophtalmiques.

Cependant, les produits décrits dans les Brevets américains n° 4 379 864 et n° 4 543 371 ne présentent pas une teneur en eau élevée ; ces polymères perdent, en effet, les propriétés mécaniques requises, notamment pour leur utilisation comme implant, lorsque la teneur en eau est élevée.

En ce qui concerne les hydrogels décrits dans la demande de Brevet européen n° 188 110, ils ne présentent pas les qualités de bonne tolérance, notamment requises pour un implant, dans la mesure où les caractéristiques ioniques et en particulier l'électronégativité du polymère ne permet pas d'envisager une bonne tolérance de celui-ci.

C'est pourquoi la Demanderesse a cherché dans une autre voie une solution au problème posé, qui est celui de fournir des hydrogels présentant une biocompatibilité élevée pour leur mise en oeuvre dans la préparation d'objets à usage médical ou chirurgical hautement fiables et en particulier des implants oculaires permettant d'obtenir une perméabilité appropriée à différentes molécules biologiques, lesdits implants, ne présentant pas, par conséquent, les inconvénients des implants connus.

Le Brevet FR 2 529 464 décrit des matériaux biocompatibles sous forme de fibres creuses ou de membranes. Ces biomatériaux sont traités par une pluralité d'étirages pour obtenir une perméabilité appropriée et présentent de ce fait, une structure différente, à savoir de membranes ou fibres creuses pour hémodialyse et/ou hémofiltration.

Le Brevet DE-A-2028956 décrit un hydrogel qui comprend de nombreux groupements ionogènes, ledit hydrogel ne présentant pas la biocompatibilité convenable recherchée par la Demanderesse.

La Demanderesse a de manière inattendue, trouvé que certains biomatériaux dans des conditions particulières, présentent des avantages importants, notamment dans le domaine des implants oculaires.

C'est, à cet égard, un but de l'invention de pourvoir à un hydrogel non réticulé à propriétés mécaniques améliorées et à teneur en eau élevée ainsi qu'à son procédé de préparation qui répondant mieux aux nécessités de la pratique que les hydrogels de l'Art antérieur, notamment en ce qu'ils ont l'avantage de conférer auxdits hydrogels un caractère d'inertie vis-à-vis des cellules biologiques.

C'est également un but de l'invention de pourvoir à des objets constitués par ou comprenant ledit hydrogel.

L'application desdits objets en chirurgie et en médecine est également un but de l'invention.

Parmi lesdits objets, on peut notamment citer les implants oculaires qui présentent outre le caractère d'inertie vis-à-vis des cellules biologiques :

- les propriétés optiques suivantes :
  . transparence parfaite à la lumière visible,
  . absorption des rayons ultra-violets à 280 nm,
  . indice de réfraction voisin de celui de la cornée.
- les propriétés physico-chimiques, telles que :
  . haute perméabilité à l'eau, au sérum physiologique, aux petites et moyennes molécules, assurant la migration des substances nutritives pour la cornée ainsi que l'ensemble des métabolites,
  . perméabilité aux gaz dissous ($0_2$, $C0_2$),
  . haute hydrophilie,
  . nature chimique dépourvue de groupements toxiques, de métaux lourds, de catalyseurs restants et de monomères libres et de solvant
  . mise en oeuvre facile,
  . stabilité dimensionnelle, notamment dans une solution de chlorure de Na à 0,9 %.
- des propriétés biologiques particulières, telles que :
  . être non-biorésorbable dans le milieu physiologique,
  . avoir une bonne résistance au vieillissement dans ce milieu, c'est-à-dire ne pas présenter d'opacification, de coloration ou de dégradation des propriétés physiques.
  . présenter une bonne tolérance tissulaire des sites d'implantations dans le stroma cornéen, sans provoquer d'altération de l'épithélium et de l'endothélium cornéens,
  . avoir si possible une faible affinité aux protéines,
  . être stérilisable et/ou re-stérilisable.

EP 0 347 267 B1

La présente invention a pour objet un hydrogel non réticulé à teneur en eau élevée, caractérisé en ce qu'il est constitué à partir d'une composition liquide de départ (D) qui comprend de 2 à 50 % d'un copolymère d'acrylonitrile et d'un comonomère oléfiniquement insaturé porteur de groupements anioniques choisis parmi les groupes sulfonate, carboxyle, phosphate, phosphonate et sulfate, le rapport molaire acrylonitrile-comonomère étant compris entre 90:10 et 100:0, un solvant dudit copolymère choisi dans le groupe qui comprend les solvants organiques polaires aprotiques et les solvants minéraux et un non-solvant dudit copolymère choisi dans le groupe qui comprend l'eau, des solutions aqueuses de sels minéraux et des solutions aqueuses de sels organiques, le rapport solvant/non-solvant étant compris entre 500:1 et 0,5:1 en poids, ledit hydrogel ayant une structure microporeuse, une capacité ionique comprise entre 0 et 500 mEq/kg de gel, une teneur hydrique comprise entre 50 et 98 % et présentant une aptitude à une déformation permanente sous contrainte, même à une température inférieure à 40 °C.

Selon un mode de réalisation avantageux dudit hydrogel, le rapport molaire acrylonitrile-comonomère est de préférence compris entre 95:5 et 99:1.

Les solvants préférés sont les solvants connus desdits copolymères, de préférence miscibles à l'eau ; plus spécifiquement, on peut citer notamment le N,N-diméthylformamide (DMF), le diméthylsulfoxyde (DMSO), la N-méthylpyrrolidone (2NMP), les solutions concentrées de chlorure de zinc ou de chlorure de calcium.

De tels solvants permettent notamment d'obtenir des hydrogels dont la structure est particulièrement appropriée à l'application opthalmologique et notamment à la kératophakie et à l'épikératophakie.

Les hydrogels selon l'invention ont une teneur résiduelle en solvant généralement inférieure à 1 % et de préférence inférieure à 0,1 %.

Selon encore un autre mode de réalisation avantageux dudit hydrogel, le non-solvant est choisi dans le groupe qui comprend l'eau, les solutions aqueuses de sel minéral approprié et les solutions aqueuses de sel organique approprié.

Selon une disposition avantageuse de ce mode de réalisation, lorsque le non-solvant est une solution aqueuse de sel, ladite solution est à une concentration comprise entre 0,5 et 5 %, de manière à obtenir dans ladite composition (D), une concentration en sel comprise entre 0,03 et 1 %, de préférence entre 0,05 et 1 %.

Les sels minéraux et organiques préférés sont le chlorure de sodium ou de potassium, l'iodate de sodium ou de potassium, le bicarbonate de sodium ou de potassium, le chlorate de sodium ou de potassium, le periodate de sodium ou de potassium, le nitrate de sodium ou de potassium, le citrate de sodium ou de potassium, le tartrate de sodium ou de potassium, l'ascorbate de sodium ou de potassium, l'acétate de sodium ou de potassium, le lactate de sodium ou de potassium.

Selon une modalité de cette disposition, la solution aqueuse de sel préférée est une solution de chlorure de sodium.

Le comonomère est avantageusement du méthallylsulfonate de sodium.

De tels hydrogels, ayant une charge électro-négative faible, ne présentent pas d'interaction avec les cellules et ont donc une tolérance nettement améliorée.

La présente invention a également pour objet un procédé de préparation de l'hydrogel non réticulé conforme à l'invention, caractérisé par les étapes suivantes :

(a) refroidissement d'une composition liquide de départ (D) comprenant de 2 à 50 % d'un copolymère d'acrylonitrile et d'un comonomère oléfiniquement insaturé porteur de groupements anioniques choisis parmi les groupes sulfonate, carboxyle, phosphate, phosphonate et sulfate, le rapport molaire acrylonitrile-comonomère étant compris entre 90:10 et 100:0, un solvant dudit copolymère choisi dans le groupe qui comprend les solvants organiques polaires aprotiques et les solvants minéraux et un non-solvant dudit copolymère choisi dans le groupe qui comprend l'eau, des solutions aqueuses de sels minéraux et des solutions aqueuses de sels organiques, le rapport solvant/non-solvant étant compris entre 500:1 et 0,5:1 en poids ;

(b) immersion du produit en cours de gélification dans un premier bain qui comprend au moins de l'eau et/ou une solution aqueuse de sel identique ou différente du non-solvant, lorsque celui-ci est lui-même une solution aqueuse de sel ;

(c) puis immersion de l'hydrogel obtenu dans au moins un deuxième bain choisi dans le groupe qui comprend l'eau et une solution aqueuse de sel à une concentration comprise entre 0,5 et 5 %, identique ou différente du non-solvant lorsque celui-ci est lui-même une solution aqueuse de sel, pour permettre la stabilisation de l'hydrogel.

Selon un mode de mise en oeuvre avantageux du procédé conforme à l'invention, le rapport molaire acrylonitrile-comonomère est de préférence compris entre 95:5 et 99:1.

3

Selon une disposition avantageuse de ce mode de mise en oeuvre, lorsque le non-solvant est une solution aqueuse de sel, il est à une concentration comprise entre 0,5 et 5 %, de manière à avoir dans ladite composition (D) une concentration en sel comprise entre 0,03 et 1 %, de préférence 0,05 et 1 %.

L'introduction d'un non-solvant et notamment de la solution aqueuse de sel approprié, dans la composition liquide de départ peut permettre d'obtenir directement un hydrogel dont la structure et les propriétés sont tout à fait adaptées à des objets à usage chirurgical ou médical.

Selon un autre mode de mise en oeuvre avantageux du procédé conforme à l'invention, l'immersion de l'étape (b) est réalisée en deux étapes, la première étape étant une immersion dans un bain d'eau à une température comprise entre 0° et -4°C pendant quelques minutes, la deuxième étape étant une immersion dans un bain d'eau à température ambiante pendant quelques minutes.

Cette immersion dans un bain d'eau froide, au cours de la gélification, a l'avantage de prévenir le froissement de surface de l'hydrogel en cours de formation, qui rendrait celui-ci notamment inutilisable en ophtalmologie ; de plus, l'immersion du gel dans un bain d'eau à température ambiante, permet le relargage complet du solvant.

Selon encore un mode de mise en oeuvre du procédé conforme à l'invention, préalablement à l'étape (c), l'hydrogel est mis sous la forme d'un objet conformé.

Selon un autre mode de mise en oeuvre du procédé conforme à l'invention, le bain de l'étape (c) est choisi dans le groupe qui comprend l'eau et une solution aqueuse de sel à une concentration comprise entre 0,5 et 5 %, identique ou différente au non-solvant lorsque celui-ci est lui-même une solution aqueuse de sel.

Selon une disposition avantageuse de ce mode de mise en oeuvre, l'immersion de l'étape (c) est réalisée à une température comprise entre la température ambiante et 70°C.

Selon encore un autre mode de mise en oeuvre avantageux, préalablement à l'étape de gélification, la composition (D) est préparée par dissolution dudit copolymère dans le solvant et le non-solvant, à une température de dissolution comprise entre 40°C et 70°C.

Selon un autre mode de mise en oeuvre avantageux de l'invention, la température de refroidissement dépend du solvant et est avantageusement comprise entre -20°C et +20°C.

L'étape (c) du procédé de préparation de l'hydrogel, à savoir la stabilisation par immersion dans une solution appropriée (sel ou eau), permet une stabilisation dimensionnelle dudit hydrogel (processus accéléré de retrait).

La présente invention a également pour objet un objet à usage médical et/ou chirurgical tel que tube, film, fil, jonc, implant et analogue, caractérisé en ce qu'il est constitué par et/ou comprend un hydrogel conforme à l'invention.

Selon un mode de réalisation avantageux de l'invention ledit objet est sous forme d'un implant oculaire.

Les implants oculaires selon l'invention trouvent application en tant qu'implant intraoculaire, épicornéen, cornéen ainsi que dans les plasties orbito-palpébrales et lacrymales.

Conformément à l'invention, ledit objet conformé peut, alors, être stérilisé par tout moyen approprié tel que les rayons ultra-violets, l'oxyde d'éthylène ou les rayonnements ionisants.

La présente invention a également pour objet un procédé de fabrication des objets conformes à l'invention, caractérisé en ce qu'un objet de forme et de dimension voulue est conformé à partir de l'hydrogel conforme à l'invention.

Selon un mode de mise en oeuvre de ce procédé, ledit objet est conformé préalablement à l'étape (c) du procédé de préparation de l'hydrogel conforme à l'invention.

Selon une disposition avantageuse de ce mode de mise en oeuvre, l'hydrogel est simultanément préparé et conformé dans un moule approprié.

Selon un autre mode de mise en oeuvre avantageux de ce procédé, ledit objet est conformé postérieurement à l'étape (c) du procédé de préparation de l'hydrogel conforme à l'invention.

Selon une disposition avantageuse de ce mode de mise en oeuvre, l'hydrogel est chauffé à une température comprise entre 50° et 90°C et moulé dans un moule approprié en deux parties.

Selon une autre disposition de ce mode de mise en oeuvre, ledit objet est conformé par usinage.

On entend par usinage, tant l'usinage mécanique que l'usinage physique (laser U.V.).

Conformément à l'invention, lorsque l'objet est conformé par moulage, le moule est avantageusement composé de deux parties définissant les surfaces concave et convexe de l'objet, caractérisé en ce que lesdites parties sont en matière plastique compatible avec le solvant.

On peut citer notamment parmi les matières plastiques utilisées le poly-oxyméthylène, les poly-oléfines, les polyamides, les silicones et le poly-tétrafluoréthylène (PTFE).

Outre les dispositions qui précèdent, l'invention comprend encore d'autres dispositions, qui ressortiront de la description qui va suivre, qui se réfère à des exemples de mise en oeuvre du procédé et de réalisation d'implants ; ainsi qu'un compte rendu d'expérimentations tant *in vitro* qu'*in vivo*.

Il doit être bien entendu, toutefois, que ces exemples de mises en oeuvre, de réalisation et de compterendu sont donnés uniquement à titre d'illustration de l'objet de l'invention, dont ils ne constituent en aucune manière une limitation.

**Exemple 1 : Préparation d'un hydrogel conforme à l'invention contenant 78 % d'eau :**

1. Composition liquide (D) de départ :
   . 9,6 % d'un copolymère 90:10 d'acrylonitrile et de méthallylsulfonate de sodium (extrait sec),
   . 86,6 % de diméthylformamide (DMF),
   . 3,8 % de NaCl à 0,9 % dans l'eau.
2. Préparation de la solution de départ :
   - On dissout le copolymère sous forme d'extrait sec dans le DMF à une température de 70°C, à l'aide d'un miniagitateur, pendant quelques minutes (5 minutes pour 2 grammes de solution, par exemple) puis on introduit le NaCl à 0,9 %. On homogénéise à l'aide d'un sonotrode ultrasonique, pendant quelques secondes.
3. Coulage :
   - On coule ladite solution de départ, formée comme précisé en 2 sur un support approprié notamment une plaque, à une température de 50°C.
4. Gélification :
   - On refroidit l'ensemble à une température d'environ -15°C et
   - On immerge immédiatement dans un bain composé de 30 % $C_2H_5OH$, 0,5 % NaCl, 69,5 % $H_2O$, à une température comprise entre - 10° à 5°C pendant 5 minutes.
   Aprés stabilisation dimensionnelle, on obtient un hydrogel qui contient 78 % d'$H_2O$.

**Exemple 2 : Préparation d'un hydrogel conforme à l'invention contenant 80 % d'eau :**

1. Composition de départ :
   . 9 % de copolymère d'acrylonitrile et de méthallylsulfonate de sodium (extrait sec),
   . 81 % de diméthylformamide (DMF),
   . 10 % de NaCl à 0,9 % dans l'eau.
2. Préparation de la solution de départ :
   Le protocole est le même que celui de l'exemple 1, à l'exception de la température de dissolution qui on ce cas est de 40°C.
   Le protocole de l'étape 3 est identique à celui de l'exemple 1.
4. Gélification :
   a) On refroidit l'ensemble à une température inférieure ou égale à 4°C pendant 20 minutes.
   b) Immersion :
   - Première étape : on immerge l'ensemble dans de l'eau à environ 0°C - 4°C, pendant 5 minutes.
   - Deuxième étape : puis l'ensemble est immergé quelques minutes dans l'eau à température ambiante, puis la membrane obtenu est séparée et immergée quelques heures dans la même eau.
5. Stabilisation : La membrane est immergée trois heures dans une solution de NaCl à 0,9 %.

**Exemple 3 : Hydrogel conforme à l'invention contenant 90 % d'eau :**

1. Composition de départ :
   . 5 % de copolymère d'acrylonitrile et de méthallylsulfonate de sodium (extrait sec),
   . 80 % de diméthylformamide (DMF),
   . 15 % de NaCl à 0,9 % dans l'eau.
2. Préparation de la solution de départ :
   Le protocole est le même que celui de l'exemple 1.
   Les étapes 3. 4. et 5. : le protocole est identique à celui de l'exemple 1.

**Exemple 4 : Hydrogel conforme à l'invention contenant 86,6 % d'eau**

- Composition liquide (D) de départ :
  . 9 % d'homopolymère (polyacrylonitrile)
  . 81,1 % de DMF
  . 9,9 % de NaCl à 0,9 % dans l'eau.
- Les étapes suivantes sont identiques à celles de l'exemple 1.

**Exemple 5 : Implant oculaire :**

La composition de départ de l'exemple 1 est coulée directement sur un moule approprié présentant les caractéristiques suivantes :

Le moule utilisé dans la présente mise en oeuvre est une combinaison de polyamide - silicone. Le moule est composé d'un support en polyamide 6,6 contenant la partie concave en élastomère silicone sans charge. La deuxième partie du moule, convexe, est une bille réalisée en élastomère-silicone de dureté Shore A 80, traitée en surface par un silicone sans charge, pour améliorer les qualités de surface de ladite bille.

La concavité de la première partie du moule a été réalisée par le procédé de "spin-casting" à l'aide d'un plateau tournant, spécialement conçu et réalisé. La vitesse de rotation de plateau, variable et affichée, permet d'obtenir une hauteur désirée d'un ménisque liquide à l'intérieur d'un cylindre posé sur ce plateau.

L'étape 4. est identique à celle de l'exemple 1.

5. Stabilisation : L'objet conformé est immergé trois heures dans une solution de NaCl à 0,9 %.

6. Stérilisation : Sept minutes à l'aide de rayons ultra-violets.

Les implants doivent être contrôlés tant du point de vue de leurs qualités optiques et de leur dimension que de leur tolérance tant *in vitro* qu'*in vivo*.

**Exemple A : Contrôle macroscopique de l'implant intraoculaire :**

Après avoir été immergé dans le sérum physiologique, l'implant est contrôlé sous une loupe binoculaire (dimensions, relief, homogénéité optique) sur fond noir, en lumière incidente. L'épaisseur de l'implant a été mesurée par un micropalpeur. Les rayons de courbure ont été mesurés à l'aide d'un lecteur de microfiches, modifié, de façon à permettre de placer sous l'objectif une cuvette contenant le liquide d'immersion et le lenticule. La projection de profil du lenticule, mise au point sur la coupe médiane, a été décalquée et les rayons mesurés avec un compas.

Les implants réalisés et testés ont les caractéristiques suivantes :
- diamètre : 6 mm,
- épaisseur centrale de l'ordre de 0,2 mm.

**Exemple B : Propriétés physicochimiques de l'implant :**

a) Retrait linéaire :

Les mesures ont été effectuées sur des échantillons ayant pour dimensions initiales : 75 x 25 x 0,8 mm.

Le retrait de l'hydrogel conforme à l'invention, est nettement plus important en présence d'une solution saline (NaCl 0,9 %), comme le montre le tableau I ci-après :

EP 0 347 267 B1

TABLEAU I

| Composition initiale du mélange : P/S/NS % | Retrait L/L . 100 (%) | | | |
|---|---|---|---|---|
| | dans $H_2O$ | % $H_2O$ | dans Sérum | % $H_2O$ |
| 5,3/83,5/11,2 | 3,9 | 94 | 27,6 | 86 |
| 7,0/82,5/10,5 | 5,2 | 91 | 27,6 | 82 |
| 9,0/81,0/10,0 | 6,6 | 88 | 24,3 | 81 |
| 11,0/80,0/9,0 | 7,9 | 86 | 21,7 | 80 |
| 13,0/79,0/8,0 | 9,2 | 85 | 18,4 | 79 |
| 19,0/75,0/6,0 | 15,7 | 78 | 17,0 | 72 |
| gélification rapide | | | | |
| 5,1/80,0/14,8 | 4,8 | 95 | 19,7 | 90 |

P : Polymère ; S : solvant ; NS : non solvant.

Ce phénomène a ses limites et le retrait ne se manifeste que jusqu'à une certaine concentration en solutés. Par exemple, le gel immergé dans la solution de NaCl à 0,9 % (sérum physiologique) présente un retrait de 21,7 %. Le même gel immergé dans une solution de NaCl à 5 % présente à peu près le même retrait d'environ 23 %.

b) perméabilité à l'eau, au sérum physiologique, aux petites et moyennes molécules :

Il s'agit d'une des propriétés fondamentales des matériaux applicables à la chirurgie réfractive cornéenne et notamment aux implants intracornéens. Cette perméabilité est une conséquence essentielle au maintien de la physiologie cornéenne dont dépend notamment la transparence de la cornée. Les flux nutritifs et métaboliques, le transport des gaz dissous (oxygène et gaz carbonique), les mouvements d'eau, ne doivent pas être entravés par la présence du lenticule.

Les implants conformes à la présente invention présentent de par leur structure particulière une très bonne perméabilité.

La perméabilité à l'eau, au sérum physiologique et à diverses substances dissoutes, a été mesurée grâce à un banc d'essai constitué d'un réservoir muni d'un agitateur. La membrane testée a été placée de façon étanche au contact d'un support de polyamide 6,6. Elle sépare donc le réservoir en deux compartiments : celui de soluté - celui du filtrat.

Toutes les mesures ont été effectuées sur des échantillons d'hydrogel en copolymère, selon l'invention, contenant 80 % d'$H_2O$, sous forme de membranes d'une épaisseur de 0,35 - 0,40 mm et d'une surface de 18 cm$^2$. Ils ont été conditionnés dans le sérum physiologique. Le gradient de pression a été maintenu à 20 cm d'$H_2O$.

La perméabilité aux petites et moyennes molécules est évaluée par le coefficient de transmittance K exprimant le rapport des concentrations de la substance dans le filtrat et dans le soluté pour un débit stable et à température ambiante.

$$K = \frac{C \ filtrat}{C \ soluté}$$

7

EP 0 347 267 B1

| Subs. | Conc. g/l | Débit $10^{-5}$ ml/mn.cm$^2$.mmHg | Coeff. de transmittance |
|---|---|---|---|
| eau | | 4,5 | - |
| NaCl (sérum ) | 0,09 | 3,5 | - |
| urée | 0,7 | 4,0 | 1 |
| créatinine | 0,05 | 2,7 | 0,98 |
| glucose | 1,1 | 2,7 | 1 |
| vitamine B$_{12}$ | $2.10^{-2}$ | 3,2 | 1 |
| albumine H | 40,0 | 1,9-1,6 | 0,4 |

c) perméabilité à l'oxygène :

Les tests ont été effectués sur des échantillons de membrane en hydrogel :
- contenant 80 % d'eau (hydrogel conforme à l'invention),
- et 70 % d'eau,

ayant une épaisseur de 0,15 - 0,25 et 0,37 mm.

La perméabilité à l'oxygène est de :

35 - $36.10^{-11}$ ml/cm$^2$/cm.s.mm Hg, pour un hydrogel à 80 % d'eau, alors qu'elle n'est que de $29.10^{-11}$ml/cm$^2$/cm.s.mm Hg, pour un hydrogel à 70 % d'eau.

d) indice de réfraction :

Il a été mesuré à l'aide du réfractomètre d'ABBE (société CARL ZEISS, W-GERMANY). Les résultats sont consignées dans le tableau II ci-après et concerne des membranes en hydrogel.

TABLEAU II

| ECHANTILLON | INDICE DE REFRACTION |
|---|---|
| Hydrogel à 85 % H$_2$0 | 1,347 |
| Hydrogel à 83 % H$_2$0 | 1,350 |
| Hydrogel à 78 % H$_2$0 | 1,368 |

e) absorption de la lumière (lumière visible et ultra-violets) :

Cette mesure a été réalisée sur des échantillons de l'hydrogel conforme à l'invention, contenant 80 % d'eau, par une méthode spectrophotométrique.

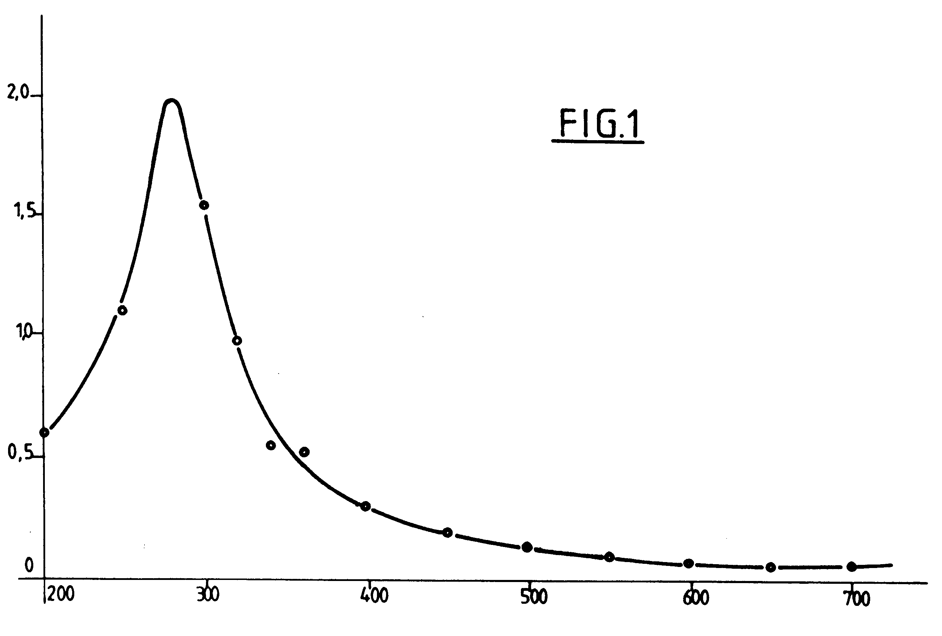

La figure 1 représente l'absorption de la lumière par l'implant.

L'axe des ordonnées représente l'absorbance :

A = - log (If/Ii),

If étant l'intensité de la lumière franchie et Ii, l'intensité de la lumière initiale.

L'axe des abscisses représente la longueur d'onde en nm.

On voit qu'il y a absorption totale à 280 nm alors qu'il n'y a pas d'absorption pour la lumière visible (400 - 700 nm).

f) tests mécaniques :

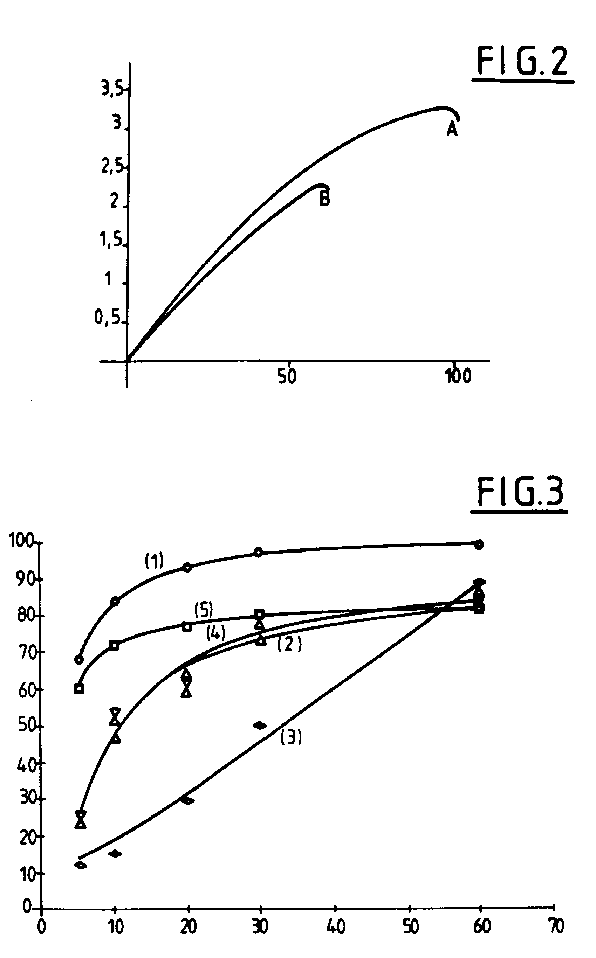

La figure 2 montre que malgré une forte teneur hydrique, la résistance à la traction est assez élevée.

L'axe des abscisses représente l'allongement en % et l'axe des ordonnées la charge en kg/cm$^2$.

La courbe A correspond à un hydrogel à 80 % d'eau.

La courbe B correspond à un hydrogel à 85 % d'eau.

**Exemple C : Evaluation "in vitro" d'un hydrogel susceptible d'être utilisé comme implant intra-cornéen :**

**- Tissu utilisé :**

Endothélium de cornée d'embryon de Poulet de 14 jours d'incubation.

8

**- Matériaux :**

. Témoin positif (toxique) : un disque filtrant (Millipore AP25 1300) imprégné d'une solution de phénol à 64 mg/l dans le milieu de culture (ph 1/100) ;

. Témoin négatif (non toxique) : Thermanose (Lux Corpo.) plastique traité pour les cultures de cellules (THX).

. Un hydrogel conforme à l'invention (80 % à 90 % $H_2O$) stérilisé aux UV et tyndalisé au moment de l'emploi (H1).

. Une membrane en polyacrylonitrile 22 $\mu$m ép. pour hémofiltration (Hospal) (H2).

**- Technique de culture :**

Le milieu de culture est le DMEM mélangé V/V à la gélose et supplémenté avec 10 % de sérum de veau foetal. Les fragments de cornée entière sont cultivés face endothéliale au contact des différents matériaux et témoins.

**- Critères d'évaluation :**

Toutes les mesures sont effectuées sur un même lot de 24 explants pour chaque type de tissu et chaque matériau, après 7 jours de culture. On mesure quantitativement les trois propriétés suivantes : multiplication, migration et adhésion cellulaire par une mesure de la surface de migration des cellules et un comptage de cellules de ce voile de migration.

. Multiplication et migration cellulaires :

Les résultats sont exprimés par la densité cellulaire en fonction de la surface de migration.

. L'adhésion cellulaire :

On utilise une technique de sensibilité des cellules à la trypsine, ce qui permet de calculer le pourcentage de cellules décrochées en fonction du temps et d'établir la courbe correspondante. A partir de cette courbe, on définit mathématiquement un indice statique d'adhésion (ISA) qui est le produit de l'aire (A) comprise entre la courbe et l'axe des x et le nombre total de cellules. Les résultats sont exprimés par l'aire de la courbe en fonction de l'ISA.

**- Résultats et interprétation :**

Les résultats sont résumés dans le tableau III ci-après :

TABLEAU III

|  | Nbre de cellules | 1 | dC | A | ISA x $10^6$ |
|---|---|---|---|---|---|
| THX | 8150±3000 | 4,9±1,8 | 1800±1000 | 4600±600 | 0,36±0,1 |
| PH1/100 | 8100±3500 | 2,6±0,8 | 3000±350 | 4900±100 | 0,4±0,1 |
| H1 | 16603±245 | 6,6±0,4 | 2500±118 | 2500±315 | 0,42±0,06 |
| H2 | 39700±7000 | 23±9 | 1730±100 | 5170±300 | 205±0,9 |

Le témoin toxique (ph 1/100) se trouve dans la zone limite du diagramme de multiplication et de migration. Il présente une légère toxicité vis-à-vis de l'endothélium de cornée et permet un attachement cellulaire très moyen.

Le témoin non toxique permet une très légère mutiplication des cellules endothéliales de la cornée qui adhèrent très moyennement à sa surface.

H2 favorise une forte migration de l'endothélium et par conséquent une faible adhésion.

H1 permet une multiplication des cellules endothéliales supérieures à celle du témoin négatif et montre un attachement cellulaire nettement supérieur à celui de tout les autres matériaux qui est très bien mis en évidence par la figure 3 (% de cellules décrochées en fonction du temps), dans laquelle l'axe des abscisses représente le temps en mn et l'axe des ordonnées représente le pourcentage de cellules décrochées. Dans cette figure, la courbe (1) correspond à H2, la courbe (2) au témoin toxique, la courbe (3) au produit conforme à l'invention, la courbe (4) au témoin non toxique et la courbe (5) à du PVC.

**Exemple D : Test de tolérance cornéenne in vivo :**

Des implants en copolymères conformes à l'invention, ont été implantés unilatéralement dans les cornées de six chats et neuf singes.

Ces implantations ont permis d'évaluer la biocompatibilité des implants et leur transparence.

La technique opératoire fait appel soit à une dissection lamellaire intra-stromale, soit, quant cela est possible, à une dissection lamellaire au micro-kératome de BARRAQUER. Le micro-kératome de BARRAQUER nécessite pour être réalisable la fixation du globe oculaire par un anneau pneumatique. Cette dissection a l'avantage de sectionner en totalité la membrane de Bowman et de permettre la déformation des couches antérieurs de la cornée sur l'implant.

1. Implantation chez le chat :

Les dissections lamellaire ont été effectuées au disciseur manuel.

. Protocole opératoire :

L'anesthésie générale est effectuée par injection sous cutanée de kétamine (environ 30 mg/kg de poids) et de l'oxybuprocaïne est systématiquement instillée dans l'oeil.

Une incision légèrement arciforme à concavité interne a été réalisée à 0,25 mm de profondeur et à 2 mm en dedans du limbe, sur environ 8 mm de longueur. Cette incision nécessite l'utilisation d'un couteau micrométrique à lame de diamant. La dissection lamellaire est continuée au disciseur Beaver et au disciseur de Paufique sur environ 9 à 10 mm.

Les implants intraoculaires sont mis en place grâce à une spatule métallique. Les sutures sont réalisées au monofilament 10/0 en polyamide et laissées en place 10 jours.

Après la mise en place des implants, un collyre contenant de la dexaméthasone et de la néomycine est instillé quotidiennement dans l'oeil opéré pendant un mois. Les chats sont examinés quotidiennement pendant toute la durée de l'expérimentation.

. Caractéristiques techniques des implants :
- la contenance hydrique des implants est de 80 % d'eau ;
- les implants ont un diamètre de 5,5 à 6,3 mm, leur épaisseur s'échelonnant de 0,20 à 0,26 mm.

. Les résultats observés chez le chat sont une bonne tolérance de la kératoprothèse, sans nécrose de la cornée réceptrice.

2. Implantation chez le primate :

. Protocole opératoire :

Neufs singes papio cynocephalus femelles (babouins) ont été opérées.

Dans trois cas, une dissection lamellaire a été effectuée selon une technique identique à celle utilisée chez le chat ; il s'agissait en effet, de singes de petite taille, ne permettant pas la fixation du globe par un anneau pneumatique. Dans les six autres cas, la dissection lamellaire au micro-kératome a été possible.

. Caractéristiques techniques des implants :
- la contenance hydrique des implants est de 60, 70 et 80 % d'eau ;
- le diamètre des implants s'échelonne de 4,8 à 7 mm ;
- l'épaisseur des implant s'échelonne de 0,16 à 0,27 mm.

. Résultats :

La transparence de la cornée et la transparence de l'implant ont été remarquables.

Ainsi que cela ressort de ce qui précède, l'invention ne se limite nullement à ceux de ses modes de réalisation, de mise en oeuvre et d'application qui viennent d'être décrits de façon plus explicite : elle en embrasse au contraire toutes les variantes qui peuvent venir à l'esprit du technicien en la matière, sans s'écarter du cadre, ni de la portée de la présente invention.

**Revendications**

1. Hydrogel non réticulé à teneur en eau élevée, caractérisé en ce qu'il est constitué à partir d'une composition liquide de départ (D) qui comprend de 2 à 50 % d'un copolymère d'acrylonitrile et d'un comonomère oléfiniquement insaturé porteur de groupements anioniques choisis parmi les groupes sulfonate, carboxyle, phosphate, phosphonate et sulfate, le rapport molaire acrylonitrile-comonomère étant compris entre 90:10 et 100:0, un solvant dudit copolymère choisi dans le groupe qui comprend les solvants organiques polaires aprotiques et les solvants minéraux et un non-solvant dudit copolymère choisi dans le groupe qui comprend l'eau, des solutions aqueuses de sels minéraux et des solutions aqueuses de sels organiques, le rapport solvant/non-solvant étant compris entre 500:1 et 0,5:1 en poids, ledit hydrogel ayant une structure microporeuse, une capacité ionique comprise entre 0 et 500 mEq/kg de gel, une teneur hydrique comprise entre 50 et 98 % et présentant une aptitude à une déformation permanente sous contrainte, même à une température inférieure à 40°C.

2. Hydrogel selon la revendication 1, caractérisé en ce que le rapport molaire acrylonitrile-comonomère est de préférence compris entre 95:5 et 99:1.

3. Hydrogel selon la revendication 1 ou la revendication 2, caractérisé en ce que lorsque le non-solvant est une solution aqueuse de sel, ladite solution est à une concentration comprise entre 0,5 et 5 %, de manière à obtenir dans ladite composition (D), une concentration en sel comprise entre 0,03 et 1 %, de préférence entre 0,05 et 1 %.

4. Hydrogel selon l'une quelconque des revendications 1 à 3, caractérisé en ce que le non-solvant est une solution de chlorure de sodium.

5. Procédé de préparation de l'hydrogel non réticulé selon l'une quelconque des revendications 1 à 4, caractérisé par les étapes suivantes :
   (a) refroidissement d'une composition liquide de départ (D) comprenant de 2 à 50 % d'un copolymère d'acrylonitrile et d'un comonomère oléfiniquement insaturé porteur de groupements anioniques choisis parmi les groupes sulfonate, carboxyle, phosphate, phosphonate et sulfate, le rapport molaire acrylonitrile-comonomère étant compris entre 90:10 et 100:0, un solvant dudit copolymère choisi dans le groupe qui comprend les solvants organiques polaires aprotiques et les solvants minéraux et un non-solvant dudit copolymère choisi dans le groupe qui comprend l'eau, des solutions aqueuses de sels minéraux et des solutions aqueuses de sels organiques, le rapport solvant/non-solvant étant compris entre 500:1 et 0,5:1 en poids ;
   (b) immersion du produit en cours de gélification dans un premier bain qui comprend au moins de l'eau et/ou une solution aqueuse de sel identique ou différente du non-solvant, lorsque celui-ci est lui-même une solution aqueuse de sel ;
   (c) puis immersion de l'hydrogel obtenu dans au moins un deuxième bain choisi dans le groupe qui comprend l'eau et une solution aqueuse de sel à une concentration comprise entre 0,5 et 5 %, identique ou différente du non-solvant lorsque celui-ci est lui-même une solution aqueuse de sel, pour permettre la stabilisation de l'hydrogel.

6. Procédé selon la revendication 5, caractérisé en ce que le rapport molaire acrylonitrile-comonomère est de préférence compris entre 95:5 et 99:1.

7. Procédé selon la revendication 5 ou la revendication 6, caractérisé en ce que lorsque le non-solvant est une solution aqueuse de sel, ladite solution est à une concentration comprise entre 0,5 et 5 %, de manière à avoir dans ladite composition (D) une concentration en sel comprise entre 0,03 et 1 %, de préférence entre 0,05 et 1 %.

8. Procédé selon l'une quelconque des revendications 5 à 7, caractérisé en ce que l'immersion de l'étape (b) est réalisée en deux étapes, la première étape étant une immersion dans un bain d'eau à une température comprise entre 0° et -4°C pendant quelques minutes, la deuxième étape étant une immersion dans un bain d'eau à température ambiante pendant quelques minutes.

9. Procédé selon l'une quelconque des revendications 5 à 8, caractérisé en ce que préalablement à l'étape (c), l'hydrogel est mis sous la forme d'un objet conformé.

10. Procédé selon la revendication 9, caractérisé en ce que l'immersion de l'étape (c) est réalisée à une température comprise entre la température ambiante et 70°C.

11. Procédé selon l'une quelconque des revendications 7 à 10, caractérisé en ce que préalablement à l'étape de gélification, la composition (D) est préparée par dissolution dudit copolymère dans le solvant et le non-solvant, à une température de dissolution comprise entre 40°C et 70°C.

12. Procédé selon l'une quelconque des revendications 5 à 11, caractérisé en ce que la température de refroidissement dépend du solvant et est avantageusement comprise entre -20°C et +20°C.

**13.** Objet à usage médical et/ou chirurgical tel que tube, film, fil, jonc, implant et analogue, caractérisé en ce qu'il est constitué par et/ou comprend un hydrogel selon l'une quelconque des revendications 1 à 4.

**14.** Objet selon la revendication 13, caractérisé en ce qu'il est sous forme d'un implant oculaire.

**15.** Objet selon la revendication 13 ou la revendication 14, caractérisé en ce qu'il peut être stérilisé par tout moyen approprié tel que les rayons ultraviolets, l'oxyde d'éthylène ou les rayonnements ionisants.

**16.** Procédé de fabrication des objets selon l'une quelconque des revendications 13 à 15, caractérisé en ce qu'un objet de forme et de dimension voulue est conformé à partir de l'hydrogel selon l'une quelconque des revendications 1 à 4.

**17.** Procédé selon la revendication 16, caractérisé en ce que ledit objet est conformé préalablement à l'étape (c) du procédé de préparation de l'hydrogel selon l'une quelconque des revendications 5 à 12.

**18.** Procédé selon la revendication 17, caractérisé en ce que l'hydrogel est simultanément préparé et conformé dans un moule approprié.

**19.** Procédé selon la revendication 16, caractérisé en ce que ledit objet est conformé postérieurement à l'étape (c) du procédé de préparation de l'hydrogel selon l'une quelconque des revendications 5 à 12.

**20.** Procédé selon la revendication 19, caractérisé en ce que l'hydrogel est chauffé à une température comprise entre 50° et 90°C et moulé dans un moule approprié en deux parties.

**21.** Procédé selon la revendication 19, caractérisé en ce que ledit objet est conformé par usinage.

**22.** Procédé selon l'une quelconque des revendications 16 à 20, caractérisé en ce que lorsque l'objet est conformé par moulage, le moule est avantageusement composé de deux parties définissant les surfaces concave et convexe de l'objet, caractérisé en ce que lesdites parties sont en matière plastique compatible avec le solvant.

**Claims**

**1.** Non-crosslinked hydrogel containing a high water content, characterized in that it is formed from a liquid starting composition (D) which comprises from 2 to 50 % of a copolymer of acrylonitrile and of an olefinically unsaturated comonomer carrying anionic groups chosen from sulphonate, carboxyl, phosphate, phosphonate and sulphate groups, the acrylonitrile to comonomer molar ratio being between 90/10 and 100/0, a solvent of the said copolymer chosen from the group which comprises aprotic polar organic solvents and inorganic solvents and a non-solvent of the said copolymer chosen from the group which comprises water, aqueous solutions of inorganic salts and aqueous solutions of organic salts, the solvent/non-solvent ratio being between 500/1 and 0.5/1 by weight, the said hydrogel having a microporous structure, an ionic capacity between 0 and 500 mEq/kg of gel, a hydric content between 50 and 98 % and capable of having the ability to permanently deform under stress, even at temperatures below 40°C.

**2.** Hydrogel according to Claim 1, characterized in that the acrylonitrile/comonomer molar ratio is preferably between 95/5 and 99/1.

**3.** Hydrogel according to Claim 1 or Claim 2, characterized in that, when the non-solvent is an aqueous salt solution, the said solution is at a concentration between 0,5 and 5 %, so as to obtain, in the said composition (D), a salt concentration between 0.03 and 1 %, preferably between 0,05 and 1 %.

**4.** Hydrogel according to any one of Claims 1 to 3, characterized in that the non-solvent is a sodium chloride solution.

**5.** Process for the preparation of the non-crosslinked hydrogel according to any one of Claims 1 to 4, characterized by the following stages:

(a) cooling a liquid starting composition (D) comprising from 2 to 50 % of a copolymer of acrylonitrile and of an olefinically unsaturated comonomer carrying anionic groups chosen from sulphonate, carboxyl, phosphate, phosphonate and sulphate groups, the acrylonitrile/comonomer molar ratio being between 90/10 and 100/0, a solvent of the said copolymer chosen from the group which comprises aprotic polar organic solvents and inorganic solvents and a non-solvent of the said copolymer chosen from the group which comprises water, aqueous solutions of inorganic salts and aqueous solutions of organic salts, the solvent/non-solvent ratio being between 500/1 and 0.5/1 by weight;

(b) immersion of the product in the course of gelling in a first bath which comprises at least water and/or an aqueous salt solution identical to or different from the non-solvent, when the latter is itself an aqueous salt solution;

(c) then immersion of the hydrogel obtained in at least one second bath chosen from the group which comprises water and an aqueous salt solution at a concentration between 0.5 and 5 %, identical to or different from the non-solvent when the latter is itself an aqueous salt solution, to enable the hydrogel to be stabilised.

6. Process according to Claim 5, characterized in that the acrylonitrile/comonomer molar ratio is preferably between 95/5 and 99/1.

7. Process according to Claim 5 or Claim 6 characterized in that, when the non-solvent is an aqueous salt solution, the said solution is at a concentration between 0.5 and 5 %, so as to have, in the said composition (D), a salt concentration between 0.03 and 1 %, preferably between 0.05 and 1 %.

8. Process according to any one of Claims 5 to 7, characterized in that the immersion of Stage (b) is carried out in two stages, the first stage being an immersion in a water bath at a temperature between 0° and -4°C for a few minutes, the second stage being an immersion in a water bath at room temperature for a few minutes.

9. Process according to any one of Claims 5 to 8, characterized in that, prior to Stage (c), the hydrogel is put into the form of a shaped object.

10. Process according to Claim 9, characterized in that the immersion of Stage (c) is carried out at a temperature between room temperature and 70°C.

11. Process according to any one of Claims 7 to 10, characterized in that, prior to the gelling stage, the composition (D) is prepared by dissolving the said copolymer in the solvent and the non-solvent, at a dissolution temperature between 40°C and 70°C.

12. Process according to any one of Claims 5 to 11, characterized in that the cooling temperature depends on the solvent and is advantageously between -20°C and +20°C.

13. Object for medical and/or surgical use, such as tube, film, thread, rod, implant and similar, characterized in that it consists of and/or comprises a hydrogel according to any one of Claims 1 to 4.

14. Object according to Claim 13, characterized in that it is in the form of an ocular implant.

15. Object according to Claim 13 or Claim 14, characterized in that it can be sterilised by any suitable means such as ultraviolet rays, ethylene oxide or ionizing radiations.

16. Process for manufacture of the objects according to any one of Claims 13 to 15, characterized in that an object of desired form and size is shaped from the hydrogel according to any one of Claims 1 to 4.

17. Process according to Claim 16, characterized in that the said object is shaped prior to Stage (c) of the process for the preparation of the hydrogel according to any one of Claims 5 to 12.

18. Process according to Claim 17, characterized in that the hydrogel is simultaneously prepared and shaped in a suitable mould.

EP 0 347 267 B1

**19.** Process according to Claim 16, characterized in that the said object is shaped subsequently to Stage (c) of the process for the preparation of the hydrogel according to any one of Claims 5 to 12.

**20.** Process according to Claim 19, characterized in that the hydrogel is heated to a temperature between 50° and 90°C and moulded in a suitable single split mould.

**21.** Process according to Claim 19, characterized in that the said object is shaped by machining.

**22.** Process according to any one of Claims 16 to 20, characterized in that, when the object is shaped by moulding, the mould is advantageously composed of two parts defining the concave and convex surfaces of the object, characterized in that the said parts are made of plastic material compatible with the solvent.

**Patentansprüche**

**1.** Nicht vernetztes Hydrogel mit erhöhtem Wassergehalt, dadurch **gekennzeichnet,** daß es gebildet ist ausgehend von einer flüssigen Ausgangszusammensetzung (D) enthaltend 2 bis 50% eines Copolymeren aus Acrylnitril und einem olefinischen ungesättigten Comonomeren, das anionische Gruppen aus den Gruppen Sulfonat, Carboxyl, Phosphat, Phosphonat und Sulfat trägt, wobei das molare Verhältnis Acrylnitril-Comonomer zwischen 90:10 und 100:0 beträgt, ein Lösungsmittel für dieses Copolymere, das aus der Gruppe umfassend organische polare aprotische Lösungsmittel und anorganische Lösungsmittel gewählt ist und ein Nichtlösungsmittel für dieses Copolymer, das aus der Gruppe umfassend Wasser, wässerige Lösungen von Mineralsalzen und wässerige Lösungen von organischen Salzen gewählt ist, wobei das Verhältnis Lösungsmittel/Nichtlösungsmittel zwischen 500:1 und 0,5:1 in Gewichtsteilen liegt und wobei dieses Hydrogel eine mikroporöse Struktur, eine ionische Kapazität zwischen 0 und 500 mEq/kg an Gel, einen Wassergehalt zwischen 50 und 98% und eine Fähigkeit zu einer permanenten Deformation unter Beanspruchung, selbst bei einer Temperatur unterhalb von 40°C hat.

**2.** Hydrogel nach Anspruch 1, dadurch **gekennzeichnet,** daß das molare Verhältnis Acrylnitril-Comonomer vorzugsweise zwischen 95:5 und 99:1 liegt.

**3.** Hydrogel nach Anspruch 1 oder 2, dadurch **gekennzeichnet,** daß wenn das Nichtlösungsmittel eine wässerige Lösung von Salz ist, dann diese Lösung bei einer Konzentration zwischen 0,5 und 5% liegt, so daß man in der Zusammensetzung (D) eine Konzentration an Salz zwischen 0,03 und 1%, vorzugsweise zwischen 0,05 und 1% hat.

**4.** Hydrogel nach irgend einem der Ansprüche 1 bis 3, dadurch **gekennzeichnet,** daß das Nichtlösungsmittel eine Lösung von Natriumchlorid ist.

**5.** Verfahren zur Herstellung des nicht vernetzten Hydrogels nach irgend einem der Ansprüche 1 bis 4, **gekennzeichnet** durch folgende Stufen:
(a) Abkühlen einer flüssigen Ausgangszusammensetzung (D), enthaltend 2 bis 50% eines Copolymeren aus Acrylnitril und einem olefinischen ungesättigten Comonomeren, das anionische Gruppen ausgewählt aus den Gruppen Sulfonat, Carboxyl, Phosphat, Phosphonat und Sulfat hat, wobei das molare Verhältnis Acrylnitril/Comonomer zwischen 90:10 und 100:0 liegt, ein Lösungsmittel für dieses Copolymere, das aus der Gruppe umfassend organische polare aprotische Lösungsmittel und anorganische Lösungsmittel gewählt ist und ein Nichtlösungsmittel für dieses Copolymer, das aus der Gruppe umfassend Wasser, wässerige Lösungen von Mineralsalzen und wässerige Lösungen von organischen Salzen gewählt ist, wobei das Verhältnis Lösungsmittel/Nichtlösungsmittel zwischen 500:1 und 0,5:1 in Gewichtsteilen liegt,
(b) Eintauchen des Produktes im Verlauf der Gelierung in ein erstes Bad, das zumindest Wasser und/oder eine wässerige Lösung von Salz enthält, die identisch oder verschieden vom Nichtlösungsmittel ist, falls dieses selbst eine wässerige Lösung von Salz ist;
(c) dann Eintauchen des erhaltenen Hydrogels in zumindest ein zweites Bad ausgewählt aus der Gruppe umfassend Wasser und einer wässerigen Lösung von Salz mit einer Konzentration zwischen 0,5 und 5%, die identisch oder verschieden ist vom Nichtlösungsmittel, falls dieses selbst eine wässerige Lösung von Salz ist, um die Stabilisierung des Hydrogels zu gestatten.

14

EP 0 347 267 B1

**6.** Verfahren nach Anspruch 5, dadurch **gekennzeichnet,** daß das molare Verhältnis Acrylnitril-Comonomer vorzugsweise zwischen 95:5 und 99:1 liegt.

**7.** Verfahren nach Anspruch 5 oder 6, dadurch **gekennzeichnet,** daß wenn das Nichtlösungsmittel eine wässerige Lösung von Salz ist, dann diese Lösung eine Konzentration zwischen 0,5 und 5% hat, so daß man in der Zusammensetzung (D) eine Konzentration an Salz zwischen 0,03 und 1%, vorzugsweise zwischen 0,05 und 1% erhält.

**8.** Verfahren nach irgend einem der Ansprüche 5 bis 7, dadurch **gekennzeichnet,** daß das Eintauchen in der Stufe (b) in zwei Stufen durchgeführt wird, wobei in der ersten Stufe ein Eintauchen in ein Bad von Wasser bei einer Temperatur zwischen 0° und -4°C während mehrerer Minuten erfolgt und in der zweiten Stufe ein Eintauchen in ein Bad von Wasser bei Umgebungstemperatur während mehrerer Minuten erfolgt.

**9.** Verfahren nach irgend einem der Ansprüche 5 bis 8, dadurch **gekennzeichnet,** daß man vor der Stufe (c) das Hydrogel in Form eines geformten Gegenstandes bringt.

**10.** Verfahren nach Anspruch 9, dadurch **gekennzeichnet,** daß das Eintauchen in der Stufe (c) bei einer Temperatur zwischen Umgebungstemperatur und 70°C durchgeführt wird.

**11.** Verfahren nach irgend einem der Ansprüche 7 bis 10, dadurch **gekennzeichnet,** daß vor der Gelierungsstufe die Zusammensetzung (D) durch Auflösen dieses Copolymeren im Lösungsmittel und im Nichtlösungsmittel bei einer Auflösungstemperatur zwischen 40°C und 70°C hergestellt wird.

**12.** Verfahren nach irgend einem der Ansprüche 5 bis 11, dadurch **gekennzeichnet,** daß die Temperatur der Abkühlung je nach dem Lösungsmittel vorteilhafterweise zwischen -20°C und +20°C liegt.

**13.** Gegenstand zur medizinischen und/oder chirurgischen Verwendung, wie ein Rohr, Film, Faden, Stab, ein Implantat und Analoge, dadurch **gekennzeichnet,** daß es aus einem Hydrogel nach irgend einem der Ansprüche 1 bis 4 besteht oder dieses enthält.

**14.** Gegenstand nach Anspruch 13, dadurch **gekennzeichnet,** daß es in Form eines Augenimplantats vorliegt.

**15.** Gegenstand nach Anspruch 13 oder 14, dadurch **gekennzeichnet,** daß es mit allen geeigneten Mitteln sterilisiert werden kann, wie UV-Strahlen, Ethylenoxid oder ionisierenden Strahlen.

**16.** Verfahren zur Herstellung von Gegenständen nach irgend einem der Ansprüche 13 bis 15, dadurch **gekennzeichnet,** daß ein Gegenstand der gewünschten Form und Abmessung ausgehend von dem Hydrogel nach irgend einem der Ansprüche 1 bis 4 geformt wird.

**17.** Verfahren nach Anspruch 16, dadurch **gekennzeichnet,** daß dieser Gegenstand vor der Stufe (c) des Verfahrens zur Herstellung des Hydrogels nach irgend einem der Ansprüche 5 bis 12 geformt wird.

**18.** Verfahren nach Anspruch 17, dadurch **gekennzeichnet,** daß das Hydrogel gleichzeitig in einer geeigneten Form hergestellt und geformt wird.

**19.** Verfahren nach Anspruch 16, dadurch **gekennzeichnet,** daß dieser Gegenstand nach Stufe (c) des Verfahrens zur Herstellung des Hydrogels nach irgend einem der Ansprüche 5 bis 12 geformt wird.

**20.** Verfahren nach Anspruch 19, dadurch **gekennzeichnet,** daß das Hydrogel auf eine Temperatur zwischen 50° und 90°C erwärmt und in einer geeigneten Form aus zwei Teilen geformt wird.

**21.** Verfahren nach Anspruch 19, dadurch **gekennzeichnet,** daß dieser Gegenstand maschinell geformt wird.

**22.** Verfahren nach irgend einem der Ansprüche 16 bis 20, dadurch **gekennzeichnet,** daß wenn der Gegenstand in einer Form geformt wird, die Form vorteilhafterweise aus zwei Teilen besteht, die die

15

konkave und konvexe Oberfläche des Gegenstandes bestimmen und weiter dadurch **gekennzeichnet,** daß diese Teile aus Kunststoffmaterial sind, das mit dem Lösungsmittel verträglich ist.

FIG.1

EP 0 347 267 B1

## FIG.2

## FIG.3